# FASCICULE DE BREVET EUROPEEN

(11) **EP 0 359 635 B1**
(45) Date de publication et mention de la délivrance du brevet: **14.12.1994**
(21) Numéro de dépôt: 89402450.4
(22) Date de dépôt: 07.09.1989
(51) Int. Cl.: A61F 5/04, A61F 5/01

(54) **Dispositif de stabilisation articulaire**
Gelenkige Stabilisierungsvorrichtung
Articulated stabilization device

(30) Priorité: 08.09.1988 FR 8811759
(43) Date de publication de la demande: 21.03.1990
(73) Titulaire: SOCIETE D'ETUDES ET DE RECHERCHES CREATIVITY Société Civile, F-13100 Aix en Provence (FR); S.A. CARTONNERIE DE BEAUSOLEIL Société Anonyme, F-83170 Brignoles (FR)
(72) Inventeur: Schlogel, Gilbert, F-83990 Saint Tropez (FR)
(74) Mandataire: Rodhain, Claude

(56) Documents cités:
- EP-A- 0 004 204
- EP-A- 0 005 615
- US-A- 2 818 063
- US-A- 3 515 131
- US-A- 3 559 640
- US-A- 3 850 167
- US-A- 4 520 806

## Description

La présente invention concerne un dispositif de stabilisation d'une articulation tel qu'une attelle ou une minerve, et notamment un dispositif destiné à être utilisé en cas d'urgence pour immobiliser momentanément une articulation, en cas de fracture.

Lorsqu'une personne se rompt un bras ou une jambe, ou toute autre articulation, il est nécessaire et recommandé d'immobiliser le membre fracturé lors du transport du lieu de l'accident vers l'hôpital ou vers le cabinet d'un médecin. En effet, si l'articulation n'est pas immobilisée, le blessé souffre atrocement et risque des complications supplémentaires.

Les secouristes ou les pompiers possèdent généralement des attelles gonflables extrêmement coûteuses. Ces attelles sont de deux types. Selon une première variante, elles sont constituées d'un manchon en matière plastique que l'on dispose autour du membre fracturé et que l'on gonfle ensuite. Selon une seconde variante, elles sont constituées d'un manchon rempli de petites billes de matière plastique, que l'on dispose autour du membre. Ensuite, on aspire l'air résiduel contenu dans le manchon et, celui-ci, grâce aux petites billes, prend la forme du contour du membre et maintient celui-ci.

Il est souvent très difficile pour les pompiers ou sauveteurs de récupérer une attelle lorsque le blessé arrive en urgence à l'hôpital. Ceci serait en effet dangereux, car il ne faut pas déplacer le membre fracturé. Du fait du prix élevé de ces attelles, ceci représente un inconvénient.

Pour les mêmes raisons, les écoles ou les centres de colonies de vacances, hésitent à acheter de telles attelles. De plus, pour être en mesure de faire face à tous les cas de fractures, ces centres devraient posséder des attelles de différentes tailles, suivant que le blessé est un tout jeune enfant ou un adolescent, voire un adulte.

En conséquence, dans la plupart des cas, le blessé est amené à l'hôpital sans attelle, ce qui est très mauvais pour son articulation.

En dehors des attelles gonflables, on connaît aussi des attelles constituées d'une planchette de bois que l'on maintient contre le membre brisé à l'aide d'un bandage. Ces attelles sont souvent "bricolées" sur le lieu de l'accident et ne sont pas très pratiques.

D'autres attelles connues sont peu pratiques, par exemple, celle décrite dans la demande de brevet européenne 4204 (CAMELIA) qui nécessite la présence de liens, et sont coûteuses à fabriquer, par exemple, celle décrite dans le brevet des Etats-Unis US-A-4 520 806 (MILLER) qui présente des éléments rapportés impliquant une ou plusieurs étapes supplémentaires de fabrication.

L'ojectif de l'invention est de réaliser un dispositif de stabilisation facile à utiliser et à fabriquer, et d'un prix de revient très faible.

Un autre objectif de l'invention est de réaliser un tel dispositif à usage unique.

Un troisième objectif de l'invention est de réaliser ce dispositif en plusieurs tailles standardisées, de façon à couvrir tous les besoins, suivant la taille de l'individu blessé.

Un quatrième objectif de l'invnetion est de réaliser ce dispositif dans un matériau permettant la circulation de l'air autour du membre, et étant radiotransparent, c'est-à-dire permettant de faire une radiographie du membre sans avoir à enlever l'attelle.

Ces objectifs ainsi que d'autres qui apparaîtront par la suite, sont atteints notamment à l'aide d'un dispositif de stabilisation articulaire constitué d'un flanc prédécoupé dans un matériau mince, celui-ci présentant, sensiblement selon son axe de symétrie, une zone longitudinale définie par au moins deux lignes de pliage, cette zone formant un fond et comportant, de part et d'autre des lignes de pliage, une aile articulée, le fond et les ailes articulées étant destinés à former une gouttière pour un segment corporel à immobiliser, caractérisé en ce qu'au moins une desdites ailes articulées est prolongée vers l'extérieur par un rabat formant couvercle, des languettes et des encoches de verrouillage étant réalisées dans lesdites ailes et coopérant de manière à solidariser ledit rabat avec l'aile opposée par emboîtage périphérique auto-bloquant avec chevauchement autour dudit segment corporel pour le stabiliser par immobilisation, sans nécessiter aucun élément additionnel.

Selon une autre caractéristique de l'invention, la zone longitudinale est divisée transversalement en deux surfaces conportant respectivement des ailes et rabats munis de languettes et encoches de verrouillage, ces deux surfaces étant articulées l'une par rapport à l'autre pour définir deux emboîtages orthogonaux correspondant aux deux parties du membre à immobiliser, respectivement la jambe et le pied, ou le bras et l'avant-bras, les ailes attenant à chacune des deux surfaces se chevauchant au niveau de l'articulation et s'auto-verrouillant par pénétration des languettes dans les encoches correspondantes.

D'autres caractéristiques et avantages de l'invention apparaîtront à la lecture de la description suivante d'un mode de réalisation préférentiel de l'invention donné à titre d'exemple illustratif et des dessins annexés dans lesquels :
- la figure 1 est une vue de dessus d'une attelle pour la jambe présentant des languettes et des fents de verrouillage ainsi qu'un couvercle, seuls les languettes et les fentes de verrouillage angulaire étant représentées selon l'invention,
- la figure 2 est une vue de dessus d'une attelle destinée à être utilisée lors d'une fracture de l'épaule,
- la figure 3 est une vue d'ensemble de l'attelle selon la figure 2, placée sur un blessé,
- la figure 4 est une vue de dessus d'un autre mode de réalisation d'une attelle pour le bras selon l'invention,
- la figure 5 est une vue de dessus d'un autre mode de réalisation d'une attelle pour la jambe selon l'invention.

Les dispositifs de stabilisation selon l'invention sont, de façon générale, découpés dans une plaque de carton mince, selon une forme adaptée à l'articulation que l'on souhaite immobiliser. Néanmoins, ils pourraient être découpés dans tout autre matériau déformable, protégeant de l'humidité ou munis de pores ou de trous destinés à permettre l'évacuation de la sueur, et radio-transparent. Ainsi, on évite que le membre ne reste à l'humidité et qu'il s'infecte, en cas de blessure, par exemple. On peut citer parmi les matériaux déformables, le carton ondulé, les matières plastiques ou synthétiques, et éventuellement les feuilles de métal ou les grillages métalliques bien que celles-ci ne permettent pas de réaliser facilement une radiographie.

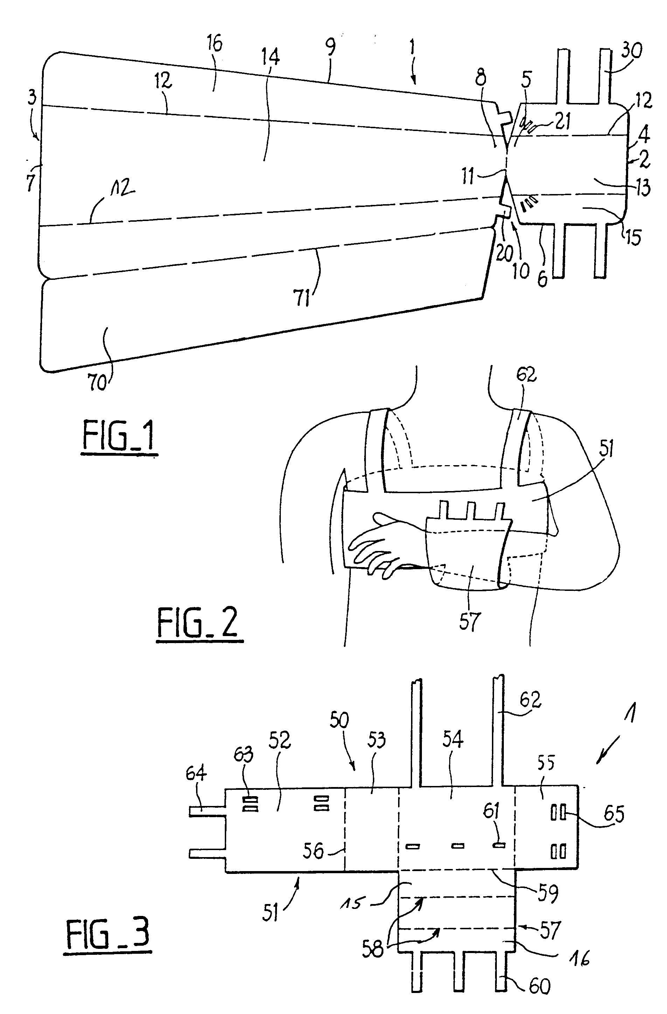

Un dispositif de stabilisation d'une jambe illustré à la figure 1 se compose d'une plaque 1 découpée de manière à former deux parties solidaires reliées par une ligne de pliage 11, de façon à être articulées l'une sur l'autre. Ces deux parties comprennent une partie plus courte 2, et une partie plus longue 3. Ces deux parties 2 et 3 présentent une forme sensiblement trapézoidale allongée et sont reliées l'une à l'autre par leur grande base. Elles pourraient aussi être rectangulaires.

Plus précisément, la partie de protection du bras 2 présente une petite base 4, une grande base 5 et deux côtés 6. La partie de protection de l'avant-bras 3 présente, elle aussi, une petite base 7, une grande base 8 et deux côtés 9. Ces deux parties 2 et 3, sont assemblées sur une partie de la longueur de leurs grandes bases respectives de façon à former deux découpes en V 10 de chaque côté, de ladite ligne de pliage 11.

Les deux parties 2 et 3 présentent en outre chacune, longitudinalement, deux lignes de pliage 12 parallèles. Ces lignes de pliage 12 partagent sensiblement en trois la largeur de chacune des parties 1 et 2 et définissent ainsi au centre un fond 13 (pour la partie 2 ; 14 pour la partie 3) et deux rebords latéraux 15 (pour la partie 2 ; 16 pour la partie 3). Il est important que l'extrémité des lignes de pliage 12 coupent l'extrémité de la ligne de pliage 11 et la partie en V de l'encoche 10, pour permettre justement un pliage facile.

Lorsque les rebords 15 (respectivement 16) sont pliés le long des lignes de pliage 12, ils forment avec le fond 13 (respectivement 14) une gouttière. En outre, les deux parties 2 et 3 articulées le long de la ligne de pliage 11 sont rapprochées l'une de l'autre de façon à former un angle variant entre environ 135° et 60°, suivant que l'on souhaite poser cette attelle sur une articulation plus ou moins pliée. Afin de maintenir en position les différentes parties pliées et notamment de maintenir l'angle d'inclinaison entre les deux parties 2 et 3, on prévoit divers moyens de maintien.

Selon une première variante de réalisation, ces moyens de maintien peuvent être constitués de deux languettes 20 disposées chacune au niveau de la découpe en V 10, le long de la grande base 5 de la partie 2, et pouvant être introduites dans des fentes 21 prévues à cet effet dans chaque rebord 16 de la partie 3. Inversement, les fentes 21 peuvent être prévues dans les rebords 16 et les languettes 20 sur les rebords 16.

Plus précisément, chaque languette 20 forme un bloc avec le rebord 15, et s'étend sensiblement perpendiculairement au centre d'un des côtés de la découpe en V 10. On prévoit de préférence trois fentes 21 dans chaque rebord 16 mais ce nombre n'est pas restrictif. Ces fentes 21 sont disposées au voisinage de la découpe en V 10 et sont ainsi en vis-à-vis des languettes 20. Ces fentes 21 sont disposées le long des rayons d'un cercle imaginaire dont le centre est situé à la pointe du V de la découpe 10. Ainsi, plus la languette 20 est insérée dans une fente 21 située loin du bord de la découpe 10, et plus l'angle entre les deux parties 2 et 3 est aigu.

De façon générale, on utilisera tout système de fixation permettant de maintenir l'inclinaison entre les deux parties, et par exemple, le collage, ou l'agrafage.

Le système permettant de faire varier l'angle d'inclinaison entre la partie de protection 2 et la partie de protection 3 est très utile car lors d'accidents, le membre peut se retrouver dans n'importe quelle position et il faut l'immobiliser dans cette position, sans le déplacer, sous peine de faire souffrir inutilement le blessé.

Comme illustré en figure 1, le dispositif de stabilisation est mis en oeuvre pour l'articulation de la cheville. La petite partie 2 sert à protéger le pied et la partie 3 sert à protéger la jambe et la cuisse, cette partie est en conséquence suffisamment longue pour entourer la longueur totale du membre inférieur. A part ces dimensions, une telle attelle est sensiblement identique à une attelle pour le bras, dans laquelle la partie 2 servirait à protéger le bras et la partie (3) à maintenir l'avant bras.

Lorsque la plaque 1 a été pliée le long des lignes de pliage 11 et 12 et que les languettes 20 ont été introduites dans les fentes 21 correspondantes, afin de former la gouttière de protection, on place l'articulation à l'intérieur de cette plaque pliée. Il est alors nécessaire de stabiliser cette plaque sur l'articulation. A cet effet, on utilise, selon un premier mode de réalisation, des liens 30 permettant de fermer la gouttière qui entoure uniquement trois faces du bras. Ces liens 30 peuvent être indépendants de la gouttière et être simplement noués autour de celle-ci. Ils peuvent être constitués par de fines cordelettes, par des bandes de papier, de tissu ou de matière plastique ou des élastiques.

Ces liens 30 peuvent aussi être formés de façon solidaire avec la plaque 1, ils sont alors réalisés dans le même matériau que celle-ci, et sont obtenus par découpage.

De préférence, on prévoit, lors de la fabrication et du prédécoupage de la plaque 1, deux liens sur chaque rebord 15 et 16, soit huit liens au total. Ces liens s'étendent sensiblement perpendiculairement à l'axe des lignes de pliage 12, et sont constitués de bandes étroites sensiblement rectangulaires.

Selon l'invention, le moyen de stabilisation de la plaque pliée sur l'articulation est constitué par un couvercle 70 (voir figure 1). Dans ce cas, on prévoit, lors du découpage le long de l'un des rebords 15, une bande sensiblement rectangulaire et de dimensions voisines de celle du fond 14. Cette bande constituant le couvercle 70 est articulée par rapport au rebord 15 ou 16 grâce à une ligne de pliage 71. Ce couvercle constitue la quatrième partie de la gouttière et est rabattu sur l'ouverture de celle-ci. Ce couvercle est maintenu en position par des moyens de fixation non représentés à la figure 1 tels que des languettes coopérant avec des fentes, ou des encoches coopérant l'une avec l'autre.

Les attelles qui viennent d'être décrites concernaient l'articulation de la cheville, mais il est bien sûr possible de reprendre le même principe pour d'autres articulations en conservant les mêmes matériaux et des moyens identiques de pliage et de fixation.

Ainsi, la figure 2 illustre une plaque 50 prédécoupée de façon à former une attelle pour l'articulation de l'épaule. En effet, en cas de fracture de l'épaule ou du bras, il est nécessaire de maintenir le bras serré contre le torse, verticalement.

La plaque 50 comprend une bande rectangulaire allongée 51 entourant le torse. Cette bande 51 se divise en deux parties sensiblement au milieu de sa longueur. Chacune de ces deux parties se divise à son tour en deux parties d'inégales longueurs.

La bande 51 est donc constituée d'une partie 52, placée contre le dos du patient (à gauche sur le dessin), d'un flanc droit 53, placé le long du côté droit du patient, d'un devant 54, destiné à être placé devant la poitrine et d'un flanc gauche 55 placé le long du côté gauche du patient.

Les deux flancs 53 et 55 sont de la même longueur. Le dos 52 et le devant 54, de longueurs identiques, sont d'une longueur sensiblement double de celle des flancs 53 et 55.

Les démarcations entre les quatre parties sont faites par trois lignes de pliage 56.

Le devant 54 présente, à sa base, une extension rectangulaire 57 constituant le support de l'avant-bras. Ce support d'avant-bras 57 est de la même longueur que le devant 54 et d'une hauteur suffisante pour entourer l'avant-bras. Il présente sensiblement, au milieu de sa hauteur, deux lignes de pliage 58 s'étendant parallèlement à l'axe longitudinal de la bande 51. En outre, le support d'avant-bras 57 est articulé par rapport à la bande 51 par une ligne de pliage 59.

Le support d'avant-bras 57 présente, à sa partie inférieure, trois languettes de fixation 60 réparties uniformément sur toute sa longueur et destinées à être placées dans des fentes 61 prévues à cet effet dans le devant 54. Le devant 54 possède en outre à sa partie supérieure, deux bretelles 62 conçues pour maintenir la bande 51 autour du corps, en passant lesdites bretelles par dessus les épaules du patient et en accrochant ces bretelles dans des fentes 63 prévues à cet effet à la partie supérieure du dos 52. L'extrémité libre latérale du dos 52 (à gauche sur le dessin) est munie de deux languettes de fixation 64 conçues pour s'engager dans deux fentes 65 prévues à cet effet dans le flanc gauche 55. Les languettes 64 et les fentes 65 sont placées en vis-à-vis et symétriquement par rapport à l'axe médian de la bande 51.

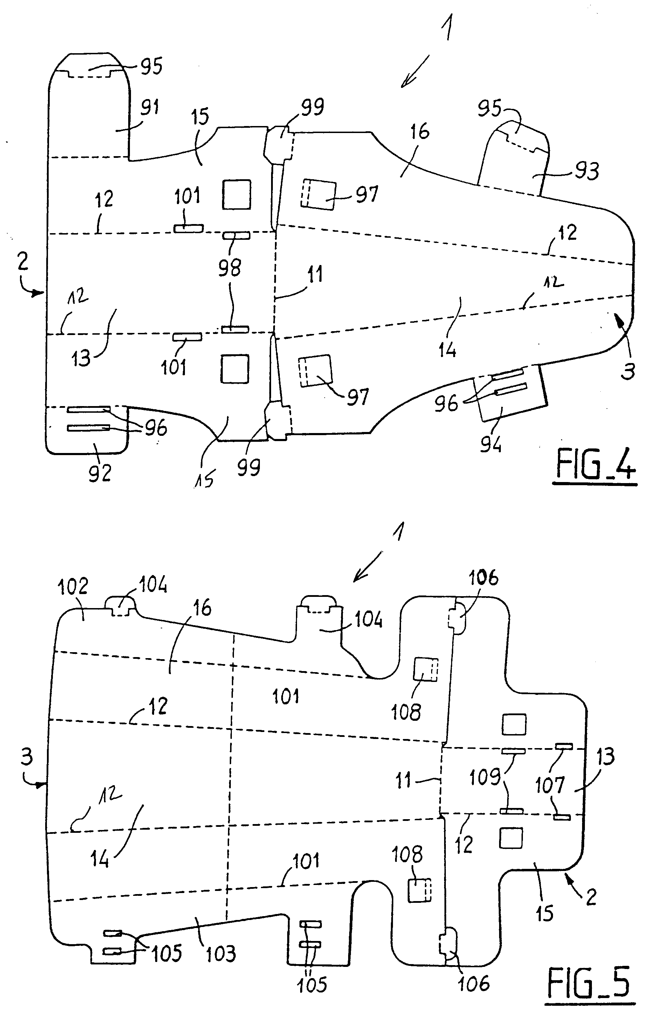

Comme illustré en figure 3, on plie les lignes de pliage 56, 58 et 59 de façon à former une coque entourant le tronc du malade et une gouttière entourant l'avant-bras. La partie de l'extension 57 munie des languettes 60 constitue un couvercle de ladite gouttière, et permet de stabiliser celle-ci sur le bras.

Ce qui vient d'être décrit pour l'attelle de l'épaule n'est qu'un mode préférentiel de réalisation, mais tout comme pour l'attelle du coude ou de la jambe on pourrait remplacer les fixations par fentes et languettes, par des fixations adhésives ou à bandes auto-aggripantes (commercialisées sous le nom de "Velcro"), ou des agrafes. De plus, même dans le mode de réalisation avec fentes ou languettes, il est possible de placer plusieurs fentes 61, 63, 65 en parallèle, au lieu d'une seule, de façon à avoir plusieurs possibilités de réglage.

La figure 4 représente une variante de réalisation d'une attelle de bras conforme à la présente invention. Sur cette figure les éléments correspondant à ceux de la figure 1 ont été repérés par les mêmes références numériques. On retrouve donc la partie 2 qui ici protége le bras, la partie 3 qui protége l'avant-bras, les lignes de pliage 11 et 12, les fonds 13 et 14 et les rebords latéraux 15 et 16.

Dans ce mode de réalisation, le verrouillage est obtenu au moyen de bandes d'épaisseur importante qui sont disposées sur les bords extérieurs des rebords ou ailes 15 et 16 en étant solidaires ce ces derniers. Ces bandes larges réalisent, lorsque l'attelle est en position d'utilisation un élément de couvercle partiel qui protège le membre. Dans le mode de réalisation représenté, la partie de protection du bras 2 et la partie de protection de l'avant bras 3 comportent chacune deux bandes 91 et 92 respectivement 93 et 94 qui constituent des demi-couvercles s'emboîtant l'un dans l'autre ; à cet effet l'une des deux bandes, les bandes 91 et 93 dans l'exemple représenté, comportent une languette 95 qui peut s'engager dans des encoches 96 aménagées dans l'autre bande à savoir les bandes 92 et 94.

Le verrouillage de l'attelle en position d'utilisation est également obtenu au moyen d'une languette 97 aménagée dans le rebord 16 et coopérant avec une encoche 98 aménagée le long de la ligne de pliage 12 de la partie 2. On prévoit également une languette 99 disposée sur la partie extérieure du rebord 16 et faisant saillie de ce dernier ; cette languette 99 coopère avec une encoche 101 aménagée également dans la partie 2 le long de la ligne de pliage 12.

La figure 5 représente une variante de réalisation d'une attelle de jambe qui comporte deux éléments en formant demi-couvercle et venant constituer un couvercle complet protégeant l'intégralité du membre. Sur cette figure, les éléments correspondant à ceux de la figure 1 ont été repérés par les mêmes références numériques.

Dans ce mode de réalisation, on a prévu dans la partie 3, deux lignes de pliage supplémentaires 101 sensiblement parallèles aux lignes de pliage 12 et qui délimitent deux éléments extérieurs 102 et 103 constituant des demi-couvercles qui réalisent, lorsque la tête est en position d'utilisation un couvercle complet de manière à entourer intégralement le membre protégé.

Les dispositifs de verrouillage sont constitués par des languettes 104 faisant saillie à l'extérieur du demi-couvercle 102 et coopérant avec des languettes 105 aménagées dans le demi-couvercle 103. Par ailleurs, on a prévu dans le rebord 16 de la partie 3 une languette 106 en saillie qui coopère avec une encoche 107 aménagée le long de la ligne de pliage 12 de la partie 2 ; une autre languette 108 découpée dans le rebord 16 et articulée sur ce dernier vient également se bloquer dans une encoche 109 ménagée le long de la ligne de pliage 12 de la partie 3.

Bien entendu, l'invention n'est pas limitée à l'exemple de réalisation ci-dessus décrit pour lequel on pourra prévoir d'autres variantes de réalisation sans pour cela sortir du cadre de l'invention comme défini dans les revendications suivantes.

## Revendications

1. Dispositif de stabilisation articulaire, constitué d'un flanc (1) prédécoupé dans un matériau mince, celui-ci présentant, sensiblement selon son axe de symétrie de stabilisation, une zone longitudinale (13, 14, 57) définie par au moins deux lignes de pliage (12, 58), cette zone formant un fond et comportant, de part et d'autre des lignes de pliage (12, 58), une aile articulée (15, 16) le fond et les ailes articulées étant destinés à former une gouttière pour un segment corporel à immobiliser, caractérisé en ce qu'au moins une desdites ailes articulées est prolongée vers l'extérieur par un rabat (54, 70, 91-94, 103, 104, 105) formant couvercle, des languettes (60, 95, 104) et des encoches (61,96, 105) de verrouillage étant réalisées dans lesdites ailes (15, 16) et coopérant de manière à solidariser ledit rabat avec l'aile opposée par emboîtage périphérique auto-bloquant avec chevauchement autour dudit segment corporel pour le stabiliser par immobilisation, sans nécessiter aucun élément additionnel.

2. Dispositif de stabilisation articulaire, selon la revendication 1, caractérisé en ce que la zone longitudinale est divisée transversalement en deux surfaces comportant respectivement des ailes et rabats munis de languettes (20, 97, 99, 106, 108) et encoches (21, 98, 101, 107, 109) de verrouillage angulaire, ces deux surfaces étant articulées l'une par rapport à l'autre pour définir deux emboîtements correspondants aux deux parties du membre à immobiliser, respectivement la jambe et le pied, ou le bras et l'avant-bras, les ailes attenant à chacune des deux surfaces se chevauchant au niveau de l'articulation et s'auto-verrouillant par pénétration des languettes de verrouillage angulaire dans les encoches correspondantes.

3. Dispositif de stabilisation articulaire selon la revendication 2, caractérisé en ce qu'il comporte plusieurs encoches de verrouillage angulaire (21) en vis-à-vis de chaque languette (20), de manière à permettre des angles d'inclinaison différents.

4. Dispositif de stabilisation articulaire selon la revendication 2, caractérisé en ce qu'il comporte une première languette de verrouillage angulaire (97) ménagée dans chaque aile (16) de manière à coopérer avec une première encoche de verrouillage angulaire (98) aménagée le long de chaque ligne de pliage (12) ainsi qu'une deuxième languette de verrouillage angulaire (99) disposée sur une partie extérieure de chaque aile (16) et faisant saillie de celle-ci et coopérant avec une encoche correspondante (101) ménagée le long de chaque ligne de pliage (12).

5. Dispositif de stabilisation articulaire selon la revendication 1, caractérisé en ce que ledit couvercle (70, 103) est solidaire par un de ses bords, de ladite plaque sur toute la longueur d'au moins une dite aile (16), et relié à celle-ci par une ligne de pliage (71, 101), le bord opposé du couvercle (70) étant maintenu solidaire de l'aile opposée par des moyens de fixation comprenant lesdites languettes (104) et encoches (105).

6. Dispositif de stabilisation articulaire selon la revendication 1, caractérisé en ce que des moyens de maintien en position pliée de la plaque ou les moyens de fixation du couvercle comportent au moins deux encoches (81) coopérant l'une avec l'autre.

7. Dispositif de stabilisation articulaire selon la revendication 1, caractérisé en ce qu'il comprend une plaque (80) de forme allongée présentant une partie centrale (86) plus large et deux extrémités rétrécies (87), en ce que l'un des bords de la partie centrale (86) présente plusieurs fentes fines (82) définissant entre elles des languettes (83), articulées autour d'une ligne de pliage (88).

8. Dispositif de stabilisation articulaire, selon la revendication 1, caractérisé en ce qu'il comprend une plaque (50) constituée d'une bande sensiblement rectangulaire (51) divisée en un dos (52), un flanc droit (53), un devant (54) constituant ledit rabat et un flanc gauche (55) séparés par des lignes de pliage (56), le devant (54) présentant une extension rectangulaire (57) constituant ladite zone longitudinale et possédant deux lignes de pliage (58) sensiblement parallèles à l'axe longitudinal de la bande (51).

## Claims

1. Device for articular stabilisation, comprising a side (1), pre-cut from a thin material, and which has, substantially along its axis of stabilisation and symmetry, a longitudinal zone (13, 14, 57) defined by at least two fold lines (12, 58), this zone forming a base and comprising an articulated flange (15, 16) on both sides of the fold lines (12, 58), the base and the articulated flanges serving to form a splint for a part of the body to be immobilised, characterised in that at least one of said articulated flanges is extended outwardly by means of a flap (54, 70, 91-94, 103, 104, 105), which forms a cover, locking tongues (60, 95, 104) and notches (61, 96, 105) being provided in said flanges (15, 16) and co-operating so as to join said flap to the opposite flange by peripheral self-locking encasement, with an overlap, around said part of the body in order to stabilise it by immobilisation, without necessitating any additional means.

2. Device for articular stabilisation according to claim 1, characterised in that the longitudinal zone is divided transversely into two surfaces comprising respectively flanges and flaps provided with angular locking tongues (20, 97, 99, 106, 108) and notches (21, 98, 101, 107, 109), these two surfaces being articulated one relative to the other to define two encasements corresponding to the two portions of the limb to be immobilised, respectively the leg and the foot, or the arm and the forearm, the flanges adjoining each of the two surfaces which overlap at the level of the joint and interlock as a result of the angular locking tongues entering the corresponding notches.

3. Device for articular stabilisation according to claim 2, characterised in that it comprises a plurality of angular locking notches (21) facing each tongue (20) so as to permit various angles of inclination.

4. Device for articular stabilization according to claim 2, characterised in that it comprises a first angular locking tongue (97), which is provided in each flange (16) so as to co-operate with a first angular locking notch (98) provided along each fold line (12), and a second angular locking tongue (99) which is disposed on an external portion of each flange (16), said tongue protruding therefrom and co-operating with a corresponding notch (101) provided along each fold line (12).

5. Device for articular stabilization according to claim 1, characterised in that said cover (70, 103) is integral, by one of its edges, with said plate over the entire length of at least one said flange (16) and is connected thereto by a fold line (71, 101), the opposite edge of the cover (70) being kept integral with the opposite flange by securing means comprising said tongues (104) and notches (105).

6. Device for articular stabilisation according to claim 1, characterised in that means for maintaining the plate in a folded position or the means for securing the cover comprise at least two notches (81) which co-operate with one another.

7. Device for articular stabilisation according to claim 1, characterised in that it comprises a plate (80) of elongate configuration having a wider central portion (86) and two narrowed ends (87), in that one of the edges of the central portion (86) has a plurality of fine slots (82), which define tongues (83) therebetween which are articulated around a fold line (88).

8. Device for articular stabilisation according to claim 1, characterised in that it comprises a plate (50) formed from a substantially rectangular strip (51), which is divided into a back (52), a right side (53), a front (54) forming said flap, and a left side (55), which are separated by fold lines (56), the front (54) having a rectangular extension (57) forming said longitudinal zone and having two fold lines (58) which extend substantially parallel to the longitudinal axis of the strip (51).

## Patentansprüche

1. Vorrichtung zur Gelenkstabilisierung, gebildet aus einem Zuschnitt (1) aus dünnem Material, welcher im wesentlichen entlang seiner Stabilisierungssymmetrieachse eine Längszone (13, 14, 57) aufweist, die durch mindestens zwei Faltlinien (12, 58) definiert ist, wobei diese Zone einen Boden bildet und auf beiden Seiten der Faltlinien (12, 58) jeweils einen Gelenkflügel (15, 16) aufweist, wobei der Boden und die Gelenkflügel dazu bestimmt sind, eine Hohlschiene für einen zu immobilisierenden Körperteil zu bilden, dadurch gekennzeichnet, daß zumindest einer der Gelenkflügel nach außen durch eine Klappe (54, 70, 91-94, 103, 104, 105) verlängert ist, die einen Deckel bildet, wobei Zungen (60, 95, 104) und Rastausnehmungen (61, 96, 105) in den Flügeln (15, 16) vorgesehen sind und in der Weise zusammenwirken, daß sie die Klappe mit dem gegenüberliegenden Flügel durch selbstverriegelnde, überlappende Umhüllung des Körperteiles fest verbinden, um diesen durch Immobilisierung zu stabilisieren, ohne irgendein zusätzliches Element zu benötigen.

2. Vorrichtung zur Gelenkstabilisierung nach Anspruch 1, dadurch gekennzeichnet, daß die Längszone quer in zwei Flächen unterteilt ist, welche jeweils mit Zungen (20, 97, 99, 106, 108) und rechteckigen Rastausnehmungen (21, 98, 101, 107, 109) versehene Flügel und Klappen aufweisen, wobei diese beiden Flächen aneinander angelenkt sind, um zwei Umhüllungen zu definieren, welche den beiden Teilen des zu immobilisierenden Körperteiles entsprechen, dem Unterschenkel und dem Fuß bzw. dem Oberarm und dem Unterarm, wobei sich die jeder der beiden Flächen angehörenden Flügel auf Höhe des Gelenkes überlappen und sich durch den Eintritt der rechteckigen Verriegelungszungen in die entsprechenden Ausnehmungen selbsttätig verriegeln.

3. Vorrichtung zur Gelenkstabilisierung nach Anspruch 2, dadurch gekennzeichnet, daß sie mehrere rechteckige Rastausnehmungen (21) gegenüber jeder Zunge (20) aufweist, um verschiedene Neigungswinkel zu gestatten.

4. Vorrichtung zur Gelenkstabilisierung nach Anspruch 2, dadurch gekennzeichnet, daß sie eine erste rechteckige Verriegelungszunge (97) in jedem Flügel (16) aufweist, die mit einer ersten rechteckigen Rastausnehmung (98) zusammenwirkt, welche entlang jeder Faltlinie (12) angeordnet ist, sowie eine zweite rechteckige Verriegelungszunge (99), die auf einem Außenabschnitt jedes Flügels (16) angeordnet ist, über diesen vorspringt und mit einer entsprechenden Ausnehmung (101) zusammenwirkt, welche entlang jeder Faltlinie (12) angeordnet ist.

5. Vorrichtung zur Gelenkstabilisierung nach Anspruch 1, dadurch gekennzeichnet, daß der Deckel (70, 103) über eine seiner Kanten mit der genannten Platte über die gesamte Länge zumindest eines Flügels (16) verbunden und an diesem über eine Faltlinie (71, 101) angelenkt ist, wobei die gegenüberliegende Kante des Deckels (70) an dem gegenüberliegenden Flügel mit Hilfe von Befestigungsmitteln gehalten ist, welche die genannten Zungen (104) und Ausnehmungen (105) umfassen.

6. Vorrichtung zur Gelenkstabilisierung nach Anspruch 1, dadurch gekennzeichnet, daß die Mittel zum Halten der Platte in gefalteter Stellung oder die Befestigungsmittel des Deckels zumindest zwei Ausnehmungen (81) aufweisen, welche miteinander zusammenwirken.

7. Vorrichtung zur Gelenkstabilisierung nach Anspruch 1, dadurch gekennzeichnet, daß sie eine langgestreckte Platte (80) umfaßt, welcher einen breiteren Mittelteil (86) und zwei schmälere Enden (87) aufweist, und daß einer der Ränder des Mittelteiles (86) mehrere schmale Schlitze (82) aufweist, die zwischeneinander Zungen (83) definieren, welche um eine Faltlinie (88) angelenkt sind.

8. Vorrichtung zur Gelenkstabilisierung nach Anspruch 1, dadurch gekennzeichnet, daß sie eine Platte (50) aufweist, welche durch ein im wesentlichen rechteckiges Band (51) gebildet ist, das in einen Rücken (52), einen rechten Seitenteil (53), einen die genannte Klappe bildenden Vorderteil (54) und einen linken Seitenteil (55) unterteilt ist, welche durch Faltlinien (56) voneinander getrennt sind, wobei der Vorderteil (54) eine rechteckige Verlängerung (57) aufweist, welche die genannte Längszone bildet und zwei Faltlinien (58) besitzt, die im wesentlichen parallel zur Längsachse des Bandes (51) verlaufen.
